(19) Europäisches Patentamt / European Patent Office / Office européen des brevets

(11) **EP 3 905 253 B1**

(12) **EUROPEAN PATENT SPECIFICATION**

(45) Date of publication and mention of the grant of the patent:
**16.10.2024 Bulletin 2024/42**

(21) Application number: **19903054.5**

(22) Date of filing: **14.01.2019**

(51) International Patent Classification (IPC):
**G16B 20/20** (2019.01)   **G16B 40/20** (2019.01)

(52) Cooperative Patent Classification (CPC):
**G16B 20/20; G16B 40/20**

(86) International application number:
**PCT/CN2019/071514**

(87) International publication number:
**WO 2020/133588 (02.07.2020 Gazette 2020/27)**

(54) **RAPID AND STABLE METHOD FOR EVALUATING INDIVIDUAL ANIMAL GENOME BREEDING VALUES**

SCHNELLES UND STABILES VERFAHREN ZUR BEWERTUNG EINZELNER TIERGENOM-ZUCHTWERTE

PROCÉDÉ RAPIDE ET STABLE D'ÉVALUATION DE VALEURS INDIVIDUELLES DE REPRODUCTION DE GÉNOME D'ANIMAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.12.2018 CN 201811620927**

(43) Date of publication of application:
**03.11.2021 Bulletin 2021/44**

(73) Proprietors:
- **Huazhong Agricultural University**
  **Wuhan, Hubei 430070 (CN)**
- **Guangzhou Yingzi Technology Co., Ltd.**
  **Guangzhou, Guangdong 510507 (CN)**

(72) Inventors:
- **ZHAO, Shuhong**
  **Wuhan, Hubei 430070 (CN)**
- **LIU, Xiaolei**
  **Wuhan, Hubei 430070 (CN)**
- **YANG, Xiang**
  **Guangzhou, Guangdong 510507 (CN)**
- **LI, Xinyun**
  **Wuhan, Hubei 430070 (CN)**
- **ZHU, Mengjin**
  **Wuhan, Hubei 430070 (CN)**
- **XIANG, Tao**
  **Wuhan, Hubei 430070 (CN)**
- **MA, Yunlong**
  **Wuhan, Hubei 430070 (CN)**
- **YU, Mei**
  **Wuhan, Hubei 430070 (CN)**
- **WANG, Zhiquan**
  **Guangzhou, Guangdong 510507 (CN)**
- **YIN, Lilin**
  **Wuhan, Hubei 430070 (CN)**

(74) Representative: **Scholz, Volker**
**Boehmert & Boehmert**
**Anwaltspartnerschaft mbB**
**Pettenkoferstrasse 22**
**80336 München (DE)**

(56) References cited:
CN-A- 103 914 632    CN-A- 105 052 729
CN-A- 106 022 005    CN-A- 107 338 321
CN-A- 107 590 364    US-A1- 2005 177 316

- **ZHE ZHANG ET AL: "Best Linear Unbiased Prediction of Genomic Breeding Values Using a Trait-Specific Marker-Derived Relationship Matrix", PLOS ONE, vol. 5, no. 9, 9 September 2010 (2010-09-09), pages e12648, XP055262731, DOI: 10.1371/journal.pone.0012648**

**(Cont. next page)**

- HE JUN ET AL: "Comparing SNP panels and statistical methods for estimating genomic breed composition of individual animals in ten cattle breeds", BMC GENETICS, vol. 19, no. 1, 1 December 2018 (2018-12-01), XP055945843, Retrieved from the Internet <URL:https://www.ncbi.nlm.nih.gov/pmc/articles/PMC6085684/pdf/12863_2018_Article_654.pdf> DOI: 10.1186/s12863-018-0654-3
- LILIN YIN: "HIBLUP User Manual", 24 September 2018 (2018-09-24), XP055945848, Retrieved from the Internet <URL:https://usermanual.wiki/Document/hiblup usermanual.170770357/help> [retrieved on 20220725]
- HENDERSON C. R.: "A Simple Method for Computing the Inverse of a Numerator Relationship Matrix Used in Prediction of Breeding Values", BIOMETRICS, vol. 32, no. 1, 1 March 1976 (1976-03-01), Hoboken, USA, pages 69, XP093137472, ISSN: 0006-341X, Retrieved from the Internet <URL:https://dx.doi.org/10.2307/2529339> DOI: 10.2307/2529339
- GILMOUR ARTHUR R. ET AL: "Average Information REML: An Efficient Algorithm for Variance Parameter Estimation in Linear Mixed Models", BIOMETRICS, vol. 51, no. 4, 1 December 1995 (1995-12-01), Hoboken, USA, pages 1440, XP093137523, ISSN: 0006-341X, Retrieved from the Internet <URL:https://dx.doi.org/10.2307/2533274> DOI: 10.2307/2533274
- ALILOO H ET AL: "Including nonadditive genetic effects in mating programs to maximize dairy farm profitability", JOURNAL OF DAIRY SCIENCE, vol. 100, no. 2, 1 January 2017 (2017-01-01), pages 1203 - 1222, XP029895889, ISSN: 0022-0302, DOI: 10.3168/JDS.2016-11261
- HENDERSON C. R.: "Estimation of Variance and Covariance Components", BIOMETRICS, vol. 9, no. 2, 1 June 1953 (1953-06-01), Hoboken, USA, pages 226, XP093137529, ISSN: 0006-341X, Retrieved from the Internet <URL:http://www.stat.yale.edu/~hz68/619/Henderson1953.pdf> DOI: 10.2307/3001853
- NEVES H H R ET AL: "Genetic variability of residual variance of production traits in Nellore beef cattle", LIVESTOCK SCIENCE, ELSEVIER, AMSTERDAM, NL, vol. 142, no. 1, 16 July 2011 (2011-07-16), pages 164 - 169, XP028104850, ISSN: 1871-1413, [retrieved on 20110726], DOI: 10.1016/J.LIVSCI.2011.07.010
- HASEMAN J. K. ET AL: "The investigation of linkage between a quantitative trait and a marker locus", BEHAVIOR GENETICS, vol. 2, no. 1, 1 March 1972 (1972-03-01), New York, pages 3 - 19, XP093137247, ISSN: 0001-8244, Retrieved from the Internet <URL:https://ibg.colorado.edu/cdrom2023/faculty/boomsma/haseman%20elston%20BG1972.pdf> DOI: 10.1007/BF01066731
- CHRISTENSEN OLE F ET AL: "Genomic prediction when some animals are not genotyped", GENETICS SELECTION EVOLUTION, vol. 42, no. 1, 1 December 2010 (2010-12-01), FR, XP093137731, ISSN: 1297-9686, Retrieved from the Internet <URL:https://gsejournal.biomedcentral.com/counter/pdf/10.1186/1297-9686-42-2.pdf> DOI: 10.1186/1297-9686-42-2
- LILIN YIN ET AL: "HIPBLUP: an integration of statistical models on the BLUP framework for efficient genetic evaluation using big genomic data", NUCLEIC ACID RESEARCH, vol. 51, no. 8, 22 February 2023 (2023-02-22)

Remarks:

The file contains technical information submitted after the application was filed and not included in this specification

**Description**

BACKGROUND OF THE INVENTION

1. Field of the Invention

The invention relates to the technical field of animal breeding, in particular to a fast and stable genomic breeding value evaluating method for animal individuals.

2. The Prior Arts

With the development of a high-density single-nucleotide polymorphisms (SNPs) genotyping technique covering the whole genome, genomic selection (prediction), as a power tool for genome statistical analysis, is widely applied to predict and estimate genetic value (breeding value) of complex traits in plant and animal breeding, increasingly in human genetics studies. Estimation of variance components is probably the most time consuming part in the process of genomic selection. Previous popular variance component estimation algorithms used in genomic selection, such as EMAI, need iterative computation and the computational complexity of each iteration is very high. Previous genomic selection programs need to compute the inverse matrix of a genomic affinity relationship matrix and the computing time is increasing rapidly with the increased genotyped sample size. The document by ALILOO H ET AL: "Including nonadditive genetic effects in mating programs to maximize dairy farm profitability",JOURNAL OF DAIRY SCIENCE, vol. 100, no. 2, 1 January 2017 (2017-01-01), pages 1203-1222, relates to including nonadditive effects to maximize dairy farm profitability and discloses the back solving additive effects and the dominant effect value of the SNP marker.

SUMMARY OF THE INVENTION

The invention aims to solve the technical problem of providing a fast and stable genomic breeding value evaluating method for animal individuals for deficiency existing in the prior art. The HE-AI algorithm based BLUP (namely best linear unbiased prediction) is called "HIBLUP", Haseman-Elston (HE) regression and Average Information (AI) algorithms based on best linear unbiased prediction (HIBLUP) program makes whole use of pedigree, phenotypic and genotypic information to predict genetic (additive and dominant) effects for each individual animal as well as effect values for each SNP marker, and the most advanced genomic breeding value prediction and variance component estimation algorithm are realized to realize genomic selection.
To solve the above-mentioned technical problem, the technical solution adopted by the invention according to claim 1 is a fast and stable genomic breeding value evaluating method for animal individuals, which is characterized in that Haseman-Elston (HE) regression and Average Information (AI) algorithms based on best linear unbiased prediction (HIBLUP) program is adopted to perform genomic breeding value prediction using phenotype, genotype and pedigree information, and a final output includes estimated genetic value of individuals, additive effect and dominant effect values of each individual, and back solution values of each genetic marker effect used on genotyping chips. The fast and stable genomic breeding value evaluating method for animal individuals specifically includes the following steps:

step 1: performing numeralization on genotypes, coding genotypes of AA, AB and BB as 0, 1, and 2, respectively, constructing a relationship A (affinity correlation) matrix and a G (state correlation) matrix among individuals based on pedigree information using a Henderson tabular method and genomic information using a VanRaden method, respectively, and then constructing a mixture correlation matrix H among the animal individuals according to information of both an A matrix and a G matrix, with following equation:

$$H = \begin{pmatrix} A_{11} - A_{12}A_{22}^{-1}A_{21} + A_{12}A_{22}^{-1}GA_{22}^{-1}A_{21} & A_{12}A_{22}^{-1}G \\ GA_{22}^{-1}A_{21} & (1-\alpha)G + \alpha A_{22} \end{pmatrix};$$

assigning the individuals to two different groups based on whether the animal individuals in the groups have genotyping information or not, wherein the group with footer "1" represents individuals that only have pedigree information but do not have genomic typing information, the group with footer "2" represents individuals that have both pedigree and genomic typing information, $A_{11}$ and $A_{22}$ represent affinity correlation matrices among individuals within group "1" and group "2", $A_{12}$ represents the affinity correlation matrices among individuals between the group "1" and the group "2", $A_{21}$ is a transpose matrix of $A_{12}$, , G represents the state correlation matrix and $\alpha$ is a relationship reconciliation percentage for combing G matrix and $A_{22}$ matrix;

step 2: deriving genetic variance and residual variance from H matrix and phenotype value using Haseman-Elston (HE) regression algorithm with following equation:

$$E(\boldsymbol{y}^T A_j \boldsymbol{y}) = \sum_{i=1}^{n} tr(A_j \boldsymbol{K_i})\sigma_i^2 + tr(A_j)\sigma_{n+1}^2 \quad ;$$

wherein E represents expected value, Y represents phenotypic value vector, $\sigma_i^2$ is an $i_{th}$ genetic variance explained by random effects, $\sigma_{n+1}^2$ is residual variance, n is number of random effects in a model, $A_j$ is a symmetric non-negative matrix, $\widehat{A_j}$ is an optimal estimation value of $A_j$, $\widehat{A}_j = \boldsymbol{H}^{-1}\boldsymbol{K}_j\boldsymbol{H}^{-1}$ and, $K_i$ is $i_{th}$ additive effect covariate matrix, and $K_j$ is $j_{th}$ additive effect covariate matrix;

step 3: setting the genetic variance and the residual variance from Haseman-Elston (HE) regression as prior values of subsequent average information (AI) iteration, then deriving the genetic variance and the residual variance using average information (AI) iteration algorithm to the convergent standard, and obtaining estimated genetic parameters, wherein the genetic parameters include the genetic variance and the residual variance;

The average information (AI) iteration algorithm can be described as parts:

a. Newton-Raphson algorithm: $$\boldsymbol{\theta}^{(k+1)} = \boldsymbol{\theta}^{(k)} - (\boldsymbol{H}es^{(k)})^{-1}\frac{\partial \boldsymbol{L}}{\partial \boldsymbol{\theta}}\boldsymbol{\theta}^{(k)} \quad ;$$

wherein $\theta$ is genetic parameters to be estimated, k is number of iterations, $\frac{\partial \boldsymbol{L}}{\partial \boldsymbol{\theta}}$ is the first derivative of maximum log-likelihood function for each parameter to be estimated, and Hes is a hessian matrix, which is the second derivative of maximum log-likelihood function for each variance;

b. Fisher scoring method, wherein the inverse matrix of Hes is replaced by its expectation matrix F, obtaining

$$\boldsymbol{\theta}^{(k+1)} = \boldsymbol{\theta}^{(k)} - (\boldsymbol{F}^{(k)})^{-1}\frac{\partial \boldsymbol{L}}{\partial \boldsymbol{\theta}}\boldsymbol{\theta}^{(k)} \quad ;$$

the AI matrix can be calculated by

$$\boldsymbol{AI} = (-\boldsymbol{H}es + \boldsymbol{F})/2 \, ;$$

and parameters are estimated with following equation:

$$\boldsymbol{\theta}^{(k+1)} = \boldsymbol{\theta}^{(k)} - (\boldsymbol{AI}^{(k)})^{-1}\frac{\partial \boldsymbol{L}}{\partial \boldsymbol{\theta}}\boldsymbol{\theta}^{(k)} \quad ;$$

step 4: using genetic parameters estimated in step 3 to solve a mixed model equation using Henderson's method 3, and getting an estimated breeding value for each individual, wherein the mixed model equation is described as:

$$\begin{bmatrix} \boldsymbol{X'X} & \boldsymbol{X'Z} \\ \boldsymbol{Z'X} & \boldsymbol{Z'Z} + \lambda\boldsymbol{K}^{-1} \end{bmatrix}\begin{bmatrix} \widehat{\boldsymbol{b}} \\ \widehat{\boldsymbol{u}} \end{bmatrix} = \begin{bmatrix} \boldsymbol{X'y} \\ \boldsymbol{Z'y} \end{bmatrix} \, , \quad V(\boldsymbol{u}) = \sigma_u^2\mathrm{K} \, , \quad V(\boldsymbol{e}) = \sigma_e^2\boldsymbol{I} \, , \quad Cov(\boldsymbol{u},\boldsymbol{e'}) = 0 \, ,$$

$$\lambda = \sigma_e^2 / \sigma_u^2$$

, X represents a design matrix corresponding to fixed effects, Z represents a design matrix corresponding to random effects, I represents a unit matrix, $K^{-1}$ represents an inverse matrix of an affinity relationship matrix, $\widehat{b}$ represents an estimated fixed effect vector, and $\widehat{u}$ represents an estimated breeding value vector;

step 5: computing the additive effects of each single nucleotide polymorphism (SNP) marker in genotyping chips with a back solving method, wherein a computing equation can be described as:

$$\hat{a} = \frac{M'K^{-1}\widehat{u}}{\sum_{i=1}^{m} 2p_i q_i} ;$$

wherein $\hat{a}$ is an additive effect value vector of SNP markers, m is number of the SNP markers, M' is the matrix for additive marker covariates, and $p_i$ and $q_i$ are allele frequency of the $i_{th}$ SNP genetic markers; and

step 6: when the genotypes of AA, AB, and BB alleles are coded as 0, 1, and 0, respectively, processing a dominant model using the same procedure of step 2 to step 5, and back solving the dominant effect value of each SNP marker.

The technical scheme is adopted to generate the following beneficial effects: according to the fast and stable genomic breeding value evaluating method for animal individuals, provided by the invention, a combined strategy of Haseman-Elston (HE) regression and Average Information (AI) algorithms is used to efficiently obtain a stable estimation of variance components, The HE-AI algorithm based BLUP (namely best linear unbiased prediction) is called "HIBLUP", Haseman-Elston (HE) regression and Average Information (AI) algorithms based on best linear unbiased prediction (HIBLUP) program makes whole use of pedigree, phenotypic and genotypic information to predict genetic (additive and dominant) effects for each individual animal as well as effect values for each SNP marker, and the most advanced genomic breeding value prediction and variance component estimation algorithm are realized to realize genomic selection.

BRIEF DESCRIPTION OF DRAWINGS

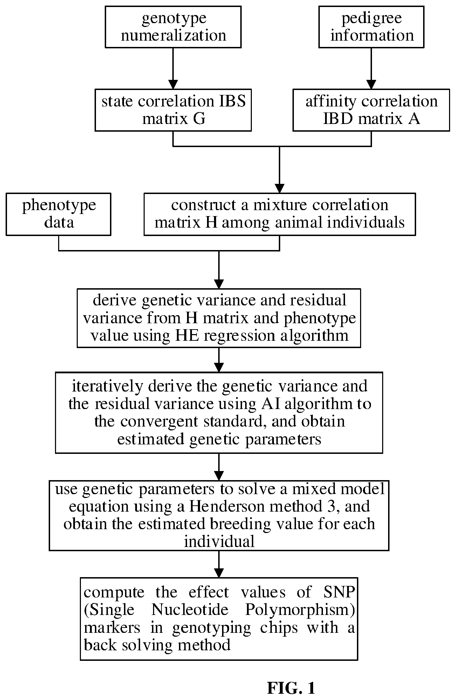

FIG. 1 shows a flow chart of a fast and stable genomic breeding value evaluating method for animal individuals according to embodiments of the invention.

DETAILED DESCRIPTION OF THE PREFERRED EMBODIMENT

The specific implementations of the invention are described in more detail below with reference to the accompanying drawings and embodiments. The following embodiments are intended to illustrate the invention, rather than to limit the scope of the invention.

As shown in FIG. 1, the method of the embodiment is described as follows.

According to the fast and stable genomic breeding value evaluating method for animal individuals, Haseman-Elston (HE) regression and Average Information (AI) algorithms based on best linear unbiased prediction (HIBLUP) program is adopted to perform genomic breeding value prediction using phenotype, genotype and pedigree information, and a final output includes estimated genetic value of individuals, additive effect and dominant effect values of each individual, and back solution values of each genetic marker effect used on genotyping chips. The fast and stable genomic breeding value evaluating method for animal individuals specifically includes the following steps:

step 1: performing numeralization on genotypes, coding genotypes of AA, AB and BB as 0, 1, and 2, respectively, constructing a relationship A (affinity correlation) matrix and a G (state correlation) matrix among individuals based on pedigree information using a Henderson tabular method and genomic information using a VanRaden method, respectively, and then constructing a mixture correlation matrix H among the animal individuals according to the information of both an A matrix and a G matrix, with following equation, wherein the matrix includes information from the A matrix and the G matrix, as shown in the below equation:

$$H = \begin{pmatrix} A_{11} - A_{12}A_{22}^{-1}A_{21} + A_{12}A_{22}^{-1}GA_{22}^{-1}A_{21} & A_{12}A_{22}^{-1}G \\ GA_{22}^{-1}A_{21} & (1-\alpha)G + \alpha A_{22} \end{pmatrix};$$

assigning the individuals to two different groups based on whether the animal individuals in the groups have genotyping information or not, wherein the group with footer "1" represents individuals that only have pedigree information but do not have genomic typing information, the group with footer "2" represents individuals that have both pedigree and genomic typing information, $A_{11}$ and $A_{22}$ represent affinity correlation matrices among individuals within group "1" and group "2", $A_{12}$ represents the affinity correlation matrices among individuals between the group "1" and the group "2", $A_{21}$ is a transpose matrix of $A_{12}$, G represents the state correlation matrix and $\alpha$ is a relationship reconciliation percentage for combing G matrix and $A_{22}$ matrix;

step 2: deriving genetic variance and residual variance from H matrix and phenotype value using Haseman-Elston (HE) regression algorithm with following equation:

$$E(y^T A_j y) = \sum_{i=1}^{n} tr(A_j K_i)\sigma_i^2 + tr(A_j)\sigma_{n+1}^2 ;$$

wherein E represents expected value, Y represents phenotypic value vector, $\sigma_i^2$ is an $i_{th}$ genetic variance explained by random effects, $\sigma_{n+1}^2$ is residual variance, n is number of random effects in a model, $A_j$ is a symmetric non-negative matrix, $\widehat{A}_j$ is an optimal estimation value of $A_j$, $\widehat{A}_j = H^{-1}K_j H^{-1}$ and, and $K_i$ is $i_{th}$ additive effect covariate matrix, and $K_j$ is $j_{th}$ additive effect covariate matrix;

step 3: setting the genetic variance and the residual variance from Haseman-Elston (HE) regression as prior values of subsequent average information (AI) iteration, then deriving the genetic variance and the residual variance using average information (AI) iteration algorithm to the convergent standard, and obtaining estimated genetic parameters, wherein the genetic parameters include the genetic variance and the residual variance;

The average information (AI) iteration algorithm can be described as parts:

a. Newton-Raphson algorithm:
$$\theta^{(k+1)} = \theta^{(k)} - (Hes^{(k)})^{-1}\frac{\partial L}{\partial \theta}\theta^{(k)} ;$$

wherein $\Theta$ is genetic parameters to be estimated, k is number of iterations, $\frac{\partial L}{\partial \theta}$ is the first derivative of maximum log-likelihood function for each parameter to be estimated, and Hes is a hessian matrix, which is the second derivative of maximum log-likelihood function for each variance;

b. Fisher scoring method, wherein the inverse matrix of Hes is replaced by its expectation matrix $F$, obtaining

$$\theta^{(k+1)} = \theta^{(k)} - (F^{(k)})^{-1}\frac{\partial L}{\partial \theta}\theta^{(k)} ;$$

the AI matrix can be calculated by;

$$AI = (-Hes + F)/2 ;$$

and parameters are estimated with following equation:

$$\boldsymbol{\theta}^{(k+1)} = \boldsymbol{\theta}^{(k)} - (\boldsymbol{AI}^{(k)})^{-1} \frac{\partial \boldsymbol{L}}{\partial \boldsymbol{\theta}} \boldsymbol{\theta}^{(k)} \quad ;$$

step 4: using genetic parameters estimated in step 3 to solve a mixed model equation using Henderson's method 3, and getting an estimated breeding value for each individual, wherein the mixed model equation is described as:

$$\begin{bmatrix} X^{'}X & X^{'}Z \\ Z^{'}X & Z^{'}Z + \lambda K^{-1} \end{bmatrix} \begin{bmatrix} \widehat{b} \\ \widehat{u} \end{bmatrix} = \begin{bmatrix} X^{'}y \\ Z^{'}y \end{bmatrix} \quad , \quad V(u) = \sigma_u^2 \mathrm{K} \quad , \quad V(e) = \sigma_e^2 I \quad , \quad Cov(u,e^{'}) = 0 \quad ,$$

$\lambda = \sigma_e^2 / \sigma_u^2$ , $X$ represents a design matrix corresponding to fixed effects, Z represents a design matrix corresponding to random effects, $I$ represents a unit matrix, $K^{-1}$ represents an inverse matrix of an affinity relationship matrix, $\widehat{b}$ represents an estimated fixed effect vector, and $\widehat{u}$ represents an estimated breeding value vector;

step 5: computing the additive effects of each single nucleotide polymorphism (SNP) marker in genotyping chips with a back solving method, wherein a computing equation can be described as:

$$\hat{a} = \frac{M^{'}K^{-1}\widehat{u}}{\sum_{i=1}^{m} 2p_i q_i} \quad ;$$

wherein $\hat{a}$ is an additive effect value vector of SNP markers, m is number of the SNP markers, $M^{'}$ is the matrix for additive marker covariates, and $p_i$ and $q_i$ are allele frequency of the $i_{th}$ SNP genetic markers; and

step 6: when the genotypes of AA, AB, and BB alleles are coded as 0, 1, and 0, respectively, processing a dominant model using the same procedure of step 2 to step 5, and back solving the dominant effect value of each SNP marker.

The application of Haseman-Elston (HE) regression and Average Information (AI) algorithms based on best linear unbiased prediction (HIBLUP) program in pig genomic selection can be used to shorten the breeding cycle (time interval), increase the selection accuracy and accelerate genetic gain for traits under selection. The application mainly includes following steps: obtaining genotype data, pedigree data and phenotype data; preparing above described datasets in Haseman-Elston (HE) regression and Average Information (AI) algorithms based on best linear unbiased prediction (HIBLUP) program input data format; running Haseman-Elston (HE) regression and Average Information (AI) algorithms based on best linear unbiased prediction (HIBLUP) program to get estimated breeding value (EBV) of each individual; computing the selection index using EBVs of multiple traits; and ranking individuals by comprehensive selection index and providing a list of selection candidates.

Finally, it should be noted that the above embodiments are merely intended to describe the technical solutions of the invention, rather than to limit the invention.

## Claims

1. A fast and stable genomic breeding value evaluating method for animal individuals carried out by a computer, wherein Haseman-Elston (HE) regression and Average Information (AI) algorithms based on best linear unbiased prediction (HIBLUP) program is adopted to perform genomic breeding value prediction using phenotype, genotype and pedigree information, and a final output includes estimated genetic value of individuals, additive effect and dominant effect values of each individual, and back solution values of each genetic marker effect used on genotyping chips; the method includes the following steps:

step 1: performing numeralization on genotypes, coding genotypes of AA, AB and BB as 0, 1, and 2, respectively, constructing a relationship A (affinity correlation) matrix and a G (state correlation) matrix among individuals based on pedigree information using a Henderson tabular method and genomic information using a VanRaden method, respectively, and then constructing a mixture correlation matrix H among the animal individuals according to information of both an A matrix and a G matrix, with following equation:

$$H = \begin{pmatrix} A_{11} - A_{2}A_{22}^{-1}A_{21} + A_{2}A_{22}^{-1}GA_{22}^{-1}A_{21} & A_{2}A_{22}^{-1}G \\ GA_{22}^{-1}A_{21} & (1-\alpha)G + \alpha A_{22} \end{pmatrix};$$

assigning the individuals to two different groups based on whether the animal individuals in the groups have genotyping information or not, wherein the group with footer "1" represents individuals that only have pedigree information but do not have genomic typing information, the group with footer "2" represents individuals that have both pedigree and genomic typing information, $A_{11}$ and $A_{22}$ represent affinity correlation matrices among individuals within group "1" and group "2", respectively, $A_{12}$ represents the affinity correlation matrices among individuals between the group "1" and the group "2", $A_{21}$ is a transpose matrix of $A_{12}$, G represents the state correlation matrix and $\alpha$ is a relationship reconciliation percentage for combing G matrix and $A_{22}$ matrix;
step 2: deriving genetic variance and residual variance from H matrix and phenotype value using Haseman-Elston (HE) regression algorithm with following equation:

$$E(\boldsymbol{y}^T A_j \boldsymbol{y}) = \sum_{i=1}^{n} tr(A_j \boldsymbol{K}_i)\sigma_i^2 + tr(A_j)\sigma_{n+1}^2;$$

wherein E represents expected value, Y represents phenotypic value vector, $\sigma_i^2$ is an $i_{th}$ genetic variance explained by random effects, $\sigma_{n+1}^2$ is residual variance, n is number of random effects in a model, $A_j$ is a symmetric non-negative matrix, $\widehat{A_j}$ is an optimal estimation value of $A_j$, $\widehat{A_j} = \boldsymbol{H}^{-1}\boldsymbol{K}_j\boldsymbol{H}^{-1}$ and, $K_i$ is $i_{th}$ additive effect covariate matrix, and $K_j$ is $j_{th}$ additive effect covariate matrix;
step 3: setting the genetic variance and the residual variance from Haseman-Elston (HE) regression as prior values of subsequent average information (AI) iteration, then deriving the genetic variance and the residual variance using average information (AI) iteration algorithm to the convergent standard, and obtaining estimated genetic parameters, wherein the genetic parameters include the genetic variance and the residual variance;
step 4: using genetic parameters estimated in step 3 to solve a mixed model equation using Henderson's method 3, and getting an estimated breeding value for each individual, wherein the mixed model equation is described as:

$$\begin{bmatrix} \boldsymbol{X}'\boldsymbol{X} & \boldsymbol{X}'\boldsymbol{Z} \\ \boldsymbol{Z}'\boldsymbol{X} & \boldsymbol{Z}'\boldsymbol{Z} + \lambda \boldsymbol{K}^{-1} \end{bmatrix} \begin{bmatrix} \widehat{\boldsymbol{b}} \\ \widehat{\boldsymbol{u}} \end{bmatrix} = \begin{bmatrix} \boldsymbol{X}'\boldsymbol{y} \\ \boldsymbol{Z}'\boldsymbol{y} \end{bmatrix}, \quad V(\boldsymbol{u}) = \sigma_u^2 K, \quad V(\boldsymbol{e}) = \sigma_e^2 I, \quad Cov(\boldsymbol{u}, \boldsymbol{e}') = 0,$$

$\lambda = \sigma_e^2 / \sigma_u^2$, X represents a design matrix corresponding to fixed effects , Z represents a design matrix corresponding to random effects , I represents a unit matrix, $K^{-1}$ represents an inverse matrix of an affinity relationship matrix, $\widehat{\boldsymbol{b}}$ represents an estimated fixed effect vector, and $\widehat{\boldsymbol{u}}$ represents an estimated breeding value vector;
step 5: computing the additive effects of each single nucleotide polymorphism (SNP) marker in genotyping chips with a back solving method, wherein a computing equation is described as:

$$\hat{\boldsymbol{a}} = \frac{\boldsymbol{M}'\boldsymbol{K}^{-1}\widehat{\boldsymbol{u}}}{\sum_{i=1}^{m} 2p_i q_i};$$

wherein $\hat{a}$ is an additive effect value vector of SNP markers, m is number of the SNP markers, $M'$ is a matrix for additive marker covariates, and $p_i$ and $q_i$ are allele frequency of $i_{th}$ SNP genetic markers; and

step 6: when genotypes of AA, AB, and BB alleles are coded as 0, 1, and 0, respectively, processing a dominant model using the same procedure of step 2 to step 5, and back solving the dominant effect value of each SNP marker,

wherein the average information (AI) iteration algorithm in step 3 is described as parts:

a. Newton-Raphson algorithm: $\theta^{(k+1)} = \theta^{(k)} - (Hes^{(k)})^{-1}\dfrac{\partial L}{\partial \theta}\theta^{(k)}$ ; wherein $\theta$ is genetic parameters

to be estimated, k is number of iterations, $\dfrac{\partial L}{\partial \theta}$ is the first derivative of maximum log-likelihood function for each parameter to be estimated, and Hes is a hessian matrix, which is the second derivative of maximum log-likelihood function for each variance;

b. Fisher scoring method, wherein the inverse matrix of Hes is replaced by its expectation matrix F, obtaining

$$\theta^{(k+1)} = \theta^{(k)} - (F^{(k)})^{-1}\frac{\partial L}{\partial \theta}\theta^{(k)} ;$$

an average information (AI) matrix is calculated by

$$AI = (-Hes + F) / 2 ;$$

and parameters are estimated with following equation:

$$\theta^{(k+1)} = \theta^{(k)} - (AI^{(k)})^{-1}\frac{\partial L}{\partial \theta}\theta^{(k)} .$$

**Patentansprüche**

1. Verfahren zur Bewertung eines schnellen und stabilen genomischen Zuchtwerts für Tierindividuen, das von einem Computer ausgeführt wird, wobei Haseman-Elston (HE)-Regressions- und Durchschnittsinformations (AI)-Algorithmen, die auf einem Best Linear Unbiased Prediction (HIBLUP)-Programm basieren, angewendet werden, um eine genomische Zuchtwertvorhersage unter Verwendung von Phänotyp-, Genotyp- und Stamminformationen durchzuführen, und eine endgültige Ausgabe einen geschätzten genetischen Wert von Individuen, additive Effekt- und dominante Effektwerte jedes Individuums und Rücklösungswerte jedes genetischen Markereffekts, der auf Genotypisierungschips verwendet wird, umfasst; wobei das Verfahren die folgenden Schritte umfasst:

Schritt 1: Durchführen einer Nummerierung an Genotypen, die Genotypen von AA, AB und BB als 0, 1 bzw. 2 codieren, Konstruieren einer Beziehungsmatrix A (Affinitätskorrelationsmatrix) und einer Matrix G (Zustandskorrelationsmatrix) unter Individuen basierend auf Stamminformationen unter Verwendung eines Henderson-Tabellenverfahrens bzw. genomischen Informationen unter Verwendung eines VanRaden-Verfahrens und dann Konstruieren einer Mischungskorrelationsmatrix H unter den Tierindividuen gemäß Informationen sowohl einer A-Matrix als auch einer G-Matrix mit der folgenden Gleichung:

$$H = \begin{pmatrix} A_{11} - A_{12}A_{22}^{-1}A_{21} + A_{12}A_{22}^{-1}GA_{22}^{-1}A_{21} & A_{12}A_{22}^{-1}G \\ GA_{22}^{-1}A_{21} & (1-\alpha)G + \alpha A_{22} \end{pmatrix} ;$$

Zuordnen der Individuen zu zwei verschiedenen Gruppen basierend darauf, ob die Tierindividuen in den Gruppen Genotypisierungsinformationen aufweisen oder nicht, wobei die Gruppe mit dem Fuß "1" Individuen darstellt, die nur Stamminformationen aufweisen, aber keine genomischen Typisierungsinformationen aufweisen, die Gruppe mit dem Fuß "2" Individuen darstellt, die sowohl Stamm- als auch genomische Typisierungsinformationen aufweisen, An und $A_{22}$ Affinitätskorrelationsmatrizen unter Individuen innerhalb der Gruppe "1" bzw. der Gruppe "2" darstellen, $A_{12}$ die Affinitätskorrelationsmatrizen unter Individuen zwischen der Gruppe "1" und der Gruppe "2" darstellt, $A_{21}$ eine transponierte Matrix von $A_{12}$ ist, G die Zustandskorrelationsmatrix darstellt und $\alpha$ ein Beziehungsabstimmungsprozentsatz für die G-Matrix und die $A_{22}$-Matrix ist;

Schritt 2: Ableiten einer genetischen Varianz und Restvarianz aus der H-Matrix und dem Phänotypwert unter Verwendung eines Haseman-Elston (HE)-Regressionsalgorithmus mit der folgenden Gleichung:

$$E(y^T A_j y) = \sum_{i=1}^{n} tr(A_j K_i)\sigma_i^2 + tr(A_j)\sigma_{n+1}^2 \; ;$$

wobei E einen erwarteten Wert darstellt, $Y$ einen Phänotypwertvektor darstellt, $\sigma_i^2$ eine $i_{te}$-genetische Varianz erklärt durch Zufallseffekte ist, $\sigma_{n+1}^2$ Restvarianz ist, n Zahl an Zufallseffekten in einem Modell ist, $A_j$ eine symmetrische Nicht-Negativ-Matrix ist, $\widehat{A_j}$ ein optimaler Schätzwert ist von $A_j$, $\widehat{A_j} = H^{-1} K_j H^{-1}$ und, $K_i$ eine $i_{te}$-Additiveffektcovariantmatrix ist, und $K_j$ $j_{te}$-Additiveffektcovariantmatrix ist;

Schritt 3: Festlegen der genetischen Varianz und der Restvarianz aus der Haseman-Elston (HE)-Regression als vorherige Werte einer nachfolgenden Durchschnittsinformations (AI)-Iteration, dann Ableiten der genetischen Varianz und der Restvarianz unter Verwendung eines Durchschnittsinformations (AI)-Iterationsalgorithmus zu dem konvergenten Standard und Erhalten geschätzter genetischer Parameter, wobei die genetischen Parameter die genetische Varianz und die Restvarianz umfassen;

Schritt 4: Verwenden von in Schritt 3 abgeschätzten genetischen Parametern, um eine Gemischtmodellgleichung unter Verwendung von Hendersons Verfahren 3 zu lösen, und Erhalten eines geschätzten Zuchtwerts für jedes Individuum, wobei die Gemischtmodellgleichung beschrieben wird als:

$$\begin{bmatrix} X'X & X'Z \\ Z'X & Z'Z + \lambda K^{-1} \end{bmatrix} \begin{bmatrix} \widehat{b} \\ \widehat{u} \end{bmatrix} = \begin{bmatrix} X'y \\ Z'y \end{bmatrix}, \quad V(u) = \sigma_u^2 K, \quad V(e) = \sigma_e^2 I, \quad Cov(u, e') = 0,$$

$\lambda = \sigma_e^2 / \sigma_u^2$, wobei X eine Designmatrix korrespondierend zu festen Effekten darstellt, Z eine Designmatrix korrespondierend zu Zufallseffekten darstellt, I eine Einheitsmatrix darstellt, $K^{-1}$ eine inverse Matrix einer Affinitätsbeziehungsmatrix darstellt, $\widehat{b}$ einen geschätzten Festeffektvektor darstellt, und $\widehat{u}$ einen geschätzten Zuchtwertfaktor darstellt;

Schritt 5: Berechnen der additiven Effekte jedes Einzelnukleotidpolymorphismus (SNP)-Markers in Genotypisierungschips mit einem Rücklösungsverfahren, wobei eine Berechnungsgleichung wie folgt beschrieben wird:

$$\hat{a} = \frac{M'K^{-1}\widehat{u}}{\sum_{i=1}^{m} 2p_i q_i} \; ;$$

wobei $\hat{a}$ ein Additiveffektwertvektor von SNP-Markern ist, m eine Zahl von SNP-Markern ist, $M'$ eine Matrix für Additivmarkercovarianten ist, und $p_i$ und $q_i$ Allelfrequenz von genetischen $i_{ten}$-SNP-Markern ist; und

Schritt 6: wenn Genotypen von AA-, AB- und BB-Allelen als 0, 1 bzw. 0 codiert sind, Verarbeiten eines dominanten Modells unter Verwendung des gleichen Verfahrens von Schritt 2 bis Schritt 5 und Rücklösen des dominanten Effektwerts jedes SNP-Markers, wobei der Durchschnittsinformations (AI)-Iterationsalgorithmus in Schritt 3 wie folgt beschrieben wird:

$$\boldsymbol{\theta}^{(k+1)} = \boldsymbol{\theta}^{(k)} - (H\mathrm{es}^{(k)})^{-1} \frac{\partial \boldsymbol{L}}{\partial \boldsymbol{\theta}} \boldsymbol{\theta}^{(k)}$$

a. Newton-Raphson-Algorithmus: ; wobei $\theta$ abzuschätzende gene-

tische Parameter ist, k Zahl an Iterationen ist, $\dfrac{\partial \boldsymbol{L}}{\partial \boldsymbol{\theta}}$ die erste Ableitung einer maximalen log-wahrscheinlichen Funktion für jeden abzuschätzenden Parameter ist, und Hes eine Hess-Matrix ist, welches die zweite Ableitung der maximalen log-wahrscheinlichen Funktion für jede Varianz ist;
b. Fisher-Scoring-Verfahren, wobei die inverse Matrix von Hes durch ihre Erwartungsmatrix $F$ ersetzt wird, erhaltend

$$\boldsymbol{\theta}^{(k+1)} = \boldsymbol{\theta}^{(k)} - (\boldsymbol{F}^{(k)})^{-1} \frac{\partial \boldsymbol{L}}{\partial \boldsymbol{\theta}} \boldsymbol{\theta}^{(k)} ;$$

wobei eine Durchschnittsinformations (AI)-Matrix berechnet wird durch

$$AI = (-H\mathrm{es} + F) / 2 ;$$

und Parameter durch folgende Gleichung geschätzt werden:

$$\boldsymbol{\theta}^{(k+1)} = \boldsymbol{\theta}^{(k)} - (AI^{(k)})^{-1} \frac{\partial \boldsymbol{L}}{\partial \boldsymbol{\theta}} \boldsymbol{\theta}^{(k)} .$$

**Revendications**

1. Procédé rapide et stable d'évaluation de valeurs individuelles de reproduction du génome animal effectué par un ordinateur, dans lequel la régression Haseman-Elston (HE) et les algorithmes d'informations moyennes (AI) basés sur la meilleure prédiction linéaire non biaisée (HIBLUP) sont adoptés pour prédire des valeurs de reproduction du génome à l'aide d'informations de phénotype, de génotype et de généalogie, et le résultat final comporte la valeur génétique estimée des espèces individuelles, les valeurs d'effets additifs et d'effets dominants de chaque espèce, et les valeurs des résolutions à rebours de chaque effet de marqueur génétique utilisé dans les puces de génotypage ; le procédé comprenant les étapes suivantes :

étape 1 : numériser les génotypes, coder les génotypes de AA, AB et BB comme 0, 1 et 2, respectivement, construire une matrice de liens A (corrélation d'affinité) et une matrice G (corrélation d'état) entre les espèces individuelles sur la base des informations de généalogie à l'aide de la méthode tabulaire de Henderson et des informations de génome à l'aide de la méthode de VanRaden, respectivement, et construire ensuite une matrice de corrélation de mélange H entre les espèces animales individuelles en fonction des informations d'une matrice A et d'une matrice G, avec l'équation suivante :

$$H = \begin{pmatrix} A_{11} - A_{12} A_{22}^{-1} A_{21} + A_{12} A_{22}^{-1} G A_{22}^{-1} A_{21} & A_{12} A_{22}^{-1} G \\ G A_{22}^{-1} A_{21} & (1-\alpha) G + \alpha A_{22} \end{pmatrix}$$

affecter les espèces individuelles à deux groupes différents selon que les animaux individuels des groupes disposent ou non d'informations de génotype, le groupe avec le pied de page "1" représentant les espèces individuelles qui disposent uniquement d'informations de généalogie mais pas d'informations de typage génomique, le groupe avec le pied de page "2" représentant les espèces individuelles qui disposent à la fois d'informations de généalogie et des informations de typage génomique, $A_{11}$ et $A_{22}$ représentent les matrices de

corrélation d'affinité entre les espèces individuelles du groupe "1" et du groupe "2", respectivement, $A_{12}$ représentant les matrices de corrélation d'affinité entre les espèces individuelles du groupe "1" et du groupe "2", $A_{21}$ désignant une matrice transposée de $A_{12}$, G représentant la matrice de corrélation d'état et $\alpha$ étant un pourcentage de réconciliation des liens pour combiner la matrice G et la matrice $A_{22}$ ;

étape 2 : dériver une variance génétique et une variance résiduelle à partir de la matrice H et de la valeur de phénotype à l'aide de l'algorithme de régression de Haseman-Elston (HE) avec l'équation suivante :

$$E(y^T A_j y) = \sum_{i=1}^{n} tr(A_j K_i)\sigma_i^2 + tr(A_j)\sigma_{n+1}^2 \quad ;$$

où E représente la valeur attendue, Y représente le vecteur des valeurs de phénotype, $\sigma_i^2$ représente une ième variance génétique expliquée par des effets aléatoires, $\sigma_{n+1}^2$ représente une variance résiduelle, n représente le nombre d'effets aléatoires dans un modèle, $A_j$ représente une matrice symétrique non négative, $\widehat{A}_j$ désigne une valeur d'estimation optimale de $A_j$, $\widehat{A}_j = H^{-1} K_j H^{-1}$ et $K_i$ désigne la ième matrice des covariables de l'effet additif et $K_j$ représente la jème matrice des covariables de l'effet additif ;

étape 3 : définir la variance génétique et la variance résiduelle de la régression de Haseman-Elston (HE) comme valeurs préalables de l'itération d'informations moyennes (AI) ultérieures, puis dériver la variance génétique et la variance résiduelle à l'aide de l'algorithme d'itération d'informations moyennes (AI) jusqu'à la norme convergente, et obtenir des paramètres génétiques estimés, les paramètres génétiques comportant la variance génétique et la variance résiduelle ;

étape 4 : utiliser les paramètres génétiques estimés à l'étape 3 pour résoudre une équation de modèle mixte en utilisant la méthode 3 de Henderson, et obtenir une valeur de reproduction estimée pour chaque espèce animale, l'équation de modèle mixte étant décrite comme suit :

$$\begin{bmatrix} X'X & X'Z \\ Z'X & Z'Z + \lambda K^{-1} \end{bmatrix} \begin{bmatrix} \hat{b} \\ \hat{u} \end{bmatrix} = \begin{bmatrix} X'y \\ Z'y \end{bmatrix}, \quad V(u) = \sigma_u^2 K, \quad V(e) = \sigma_e^2 I, \quad Cov(u, e') = 0,$$

$\lambda = \sigma_e^2 / \sigma_u^2$, X représente une matrice de conception correspondant à des effets fixes, Z représente une matrice de conception correspondant à des effets aléatoires, I représente une matrice d'unités, $K^{-1}$ représente une matrice inverse d'une matrice de liens d'affinité, $\hat{b}$ représente un vecteur d'effets fixes estimés et $\hat{u}$ représente un vecteur de valeurs de reproduction estimées ;

étape 5: calculer les effets additifs de chaque marqueur de polymorphisme de nucléotides simples (SNP) dans les puces de génotypage à l'aide d'une méthode de résolution à rebours, dans laquelle une équation de calcul est décrite comme suit :

$$\hat{a} = \frac{M'K^{-1}\hat{u}}{\sum_{i=1}^{m} 2p_i q_i} \quad ;$$

où $\hat{a}$ représente un vecteur de valeur d'effets additifs des marqueurs SNP, m désigne le nombre de marqueurs SNP, M' représente une matrice destinée aux covariables des marqueurs additifs et $p_i$ et $q_i$ désignent la fréquence des allèles du ième des marqueurs génétiques SNP ; et

étape 6 : lorsque les génotypes des allèles AA, AB et BB sont codés comme 0, 1 et 0, respectivement, traiter un modèle dominant en utilisant la même procédure de l'étape 2 à l'étape 5 et résoudre à rebours la valeur de l'effet dominant de chaque marqueur SNP,

l'algorithme d'itération des informations moyennes (AI) à l'étape 3 étant décrit comme suit :

$$\boldsymbol{\theta}^{(k+1)} = \boldsymbol{\theta}^{(k)} - (H\mathrm{es}^{(k)})^{-1} \frac{\partial \boldsymbol{L}}{\partial \boldsymbol{\theta}} \boldsymbol{\theta}^{(k)}$$

a. Algorithme de Newton-Raphson : ; où $\theta$ représente les paramètres

génétiques à estimer, k représente le nombre d'itérations, $\dfrac{\partial \boldsymbol{L}}{\partial \boldsymbol{\theta}}$ est la première dérivée de la fonction de log-vraisemblance maximale pour chaque paramètre à estimer, et Hes est une matrice hessienne, qui est la deuxième dérivée de la fonction de log-vraisemblance maximale pour chaque variance ;
b. Méthode de notation de Fisher, dans laquelle la matrice inverse de Hes est remplacée par sa matrice d'espérance $F$, ce qui permet d'obtenir

$$\boldsymbol{\theta}^{(k+1)} = \boldsymbol{\theta}^{(k)} - (\boldsymbol{F}^{(k)})^{-1} \frac{\partial \boldsymbol{L}}{\partial \boldsymbol{\theta}} \boldsymbol{\theta}^{(k)} ;$$

une matrice d'informations moyennes (AI) est calculée comme suit

$$AI = (-H\mathrm{es} + \boldsymbol{F}) / 2 ;$$

et les paramètres sont estimés à l'aide de l'équation suivante :

$$\boldsymbol{\theta}^{(k+1)} = \boldsymbol{\theta}^{(k)} - (\boldsymbol{AI}^{(k)})^{-1} \frac{\partial \boldsymbol{L}}{\partial \boldsymbol{\theta}} \boldsymbol{\theta}^{(k)} .$$

```
┌─────────────────────┐              ┌─────────────────────┐
│      genotype       │              │      pedigree       │
│   numeralization    │              │    information      │
└─────────────────────┘              └─────────────────────┘
           │                                    │
           ▼                                    ▼
┌─────────────────────┐              ┌─────────────────────┐
│ state correlation IBS│             │ affinity correlation│
│      matrix G       │              │     IBD matrix A    │
└─────────────────────┘              └─────────────────────┘
           │                                    │
           └─────────────────┬──────────────────┘
                             │
┌───────────────┐   ┌──────────────────────────────────────┐
│   phenotype   │   │    construct a mixture correlation    │
│     data      │   │   matrix H among animal individuals   │
└───────────────┘   └──────────────────────────────────────┘
        │                         │
        └────────────┬────────────┘
                     ▼
        ┌──────────────────────────────────────┐
        │  derive genetic variance and residual │
        │ variance from H matrix and phenotype  │
        │   value using HE regression algorithm │
        └──────────────────────────────────────┘
                          │
                          ▼
        ┌──────────────────────────────────────┐
        │ iteratively derive the genetic variance and │
        │  the residual variance using AI algorithm to │
        │     the convergent standard, and obtain      │
        │        estimated genetic parameters          │
        └──────────────────────────────────────┘
                          │
                          ▼
        ┌──────────────────────────────────────┐
        │ use genetic parameters to solve a mixed model │
        │   equation using a Henderson method 3, and    │
        │  obtain the estimated breeding value for each  │
        │                individual                      │
        └──────────────────────────────────────┘
                          │
                          ▼
        ┌──────────────────────────────────────┐
        │ compute the effect values of SNP      │
        │ (Single  Nucleotide   Polymorphism)   │
        │ markers in genotyping chips with a    │
        │ back solving method                   │
        └──────────────────────────────────────┘
```

**FIG. 1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

• **ALILOO H et al.** Including nonadditive genetic effects in mating programs to maximize dairy farm profitability. *JOURNAL OF DAIRY SCIENCE,* 01 January 2017, vol. 100 (2), 1203-1222